# EUROPEAN PATENT APPLICATION

(11) **EP 2 085 089 A1**
(43) Date of publication of application: **05.08.2009**
(21) Application number: 08101213.0
(22) Date of filing: 01.02.2008
(51) Int. Cl.: A61K 31/6615, A61P 3/04, A61P 25/30

(54) **Phospholipids Containing omega-3-Fatty Acids for the Treatment of Overweight, Obesity and Addictive Behavior**

(71) Applicant: KTB-Tumorforschungs GmbH, 79106 Freiburg (DE)
(72) Inventor: Massing, Ulrich, Prof. Dr., 79249, Merzhausen (DE); Taylor, Lenka, 79106, Freiburg (DE)
(74) Representative: Helbing, Jörg

(57) **Abstract**

The invention relates to the use of phospholipids containing ω-3-fatty acids and/or containing low amounts of ω-6 fatty acids, notably phospholipids of marine origin, for the treatment of overweight, obesity and addictive behavior.

## Description

The invention relates to the use of phospholipids containing ω-3-fatty acids and/or containing low amounts of ω-6 fatty acids, notably phospholipids of marine origin, for the treatment of overweight, obesity and addictive behavior.

### Background of the Invention

Overweight and obesity are regarded as a chronic disease with decreased quality of life and high risk of morbidity and mortality. It is defined as a body weight gain caused by abnormally increased body fat mass (body mass index (BMI) = weight in kg/(height*height in m) > 25 is overweight; ≥ 30 is obese). The cause is a disproportion of energy uptake and energy consumption, the energy balance is positive over a prolonged period of time.

Appetite and eating behavior are regulated by various internal messengers and neurotransmitters. The feeling of satiation is caused by expansion of the stomach which stimulates the *nervus vagus*, a big parasympathic nerve that passes the stimulus on to the hypothalamus. Different neurotransmitters with anorectic effect are activated, for example Noradrenaline, Serotonine and neuropeptides as Glucagon-like Peptide (GLP-1) and α-Melanocyte-stimulating hormone (α-MSH). After maximum satiation and interruption of intake of food, the amount of the anorectic neurotransmitters decreases again and eventually a feeling of hunger starts to develop. Again, the primary stimulus comes from the stomach, the hormone Ghreline activates orexigenic neurotransmitters in the hypothalamus, for example Neuropeptide Y (NPY) and Agouti-Related Peptide (AgRP), antagonizing α-MSH, as well as Anandamide, a endogenous agonist of the Cannabinoid-1-receptor (CB1).

CB1 receptors are widespread in the mammalian brain. The main agonist known is Tetrahydrocannabinol (THC), a lipophilic component of the plant *cannabis sativa*. THC is well known to have an - among others - appetite stimulating effect. The synthetic analogon Nabilon is used to stimulate appetite in patients suffering from AIDS- or tumor-induced cachexia. Another effect of cannabinoid receptor stimulation is the activation of dopaminergic pathways in the mesolimbic system, the so called "reward system" which is known to cause addictive behavior and "craving" in animals and humans. The stimulation of these reward mechanisms leads to an excessive intake of food, preferentially high in fat and dense in energy as well as craving for nicotine or alcohol in addicted people.

To make use of the described role of the CB1 receptor in the regulation of food intake, a selective antagonist has recently been developed as treatment of obesity in humans. The substance is known as Rimonabant and obtained approval as anti-obesity, appetite reducing drug under the name Acomplia^{®} in Europe in 2006. Its effect is the blocking of CB1 receptors, so that endogenous agonists can not stimulate the receptor and the orexigenic and/or reward signal will not be transmitted. Apart from its use as anti-obesity drug, rimonabant has been evaluated in several trials as supporting drug for smoking cessation. A systematic review by the Cochrane Collaboration lead to the result that rimonabant may increase the odds of quitting approximately 1,5 fold (Cahill K., Ussher M.; Cochrane Database Syst Rev 2007(4):CD005353*)*. The drug has not been approved by the FDA in the USA as a recent meta-analysis of clinical trials with Rimonabant has resulted in a high incidence (OR = 2.5; p = 0.01) of depressive mood disorders caused by the drug in obese patients (Christensen R. et al.; Lancet 2007, 370(9600):1706-1713). Another substance with similar action, Taranabant, is being examined in clinical trials but also shows psychiatric effects (Addy C. et al., Cell Metab 2008, 7(1):68-78).

The endogenous CB-receptor full agonists known today are Arachidonylethanolamide (Anandamide) and 2-Arachidonylglycerol (2-AG). Both derive from Arachidonic Acid (AA), an Omega-6-fatty acid which is taken up in high amounts with food from mammalian origin, like meat and eggs. AA is essential for the human organism as it is an important precursor for lipid messengers, not only Anandamide and 2-AG, but also prostaglandins, for example PGE2, and leukotrienes, which are responsible for inflammatory processes. PGE2 is the most prominent member of the prostaglandin family, this messenger is responsible for the initiation and prolongation of the inflammation response as well as pain and metastatic processes. In order for AA to be transformed into these lipid messengers, it needs to be cleaved enzymatically out of a phospholipid molecule building the cellular membrane.

Phospholipids (PL) are a main component of cellular membranes. They consist of a hydrophilic head group which is connected to the hydrophobic molecule part via a phosphate group. Their structure is as follows: 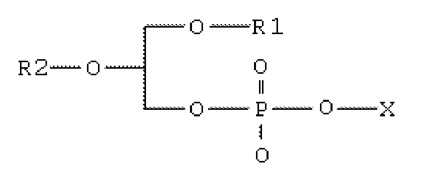 wherein X can be among others choline, serine, glycerol, ethanolamine "PG (Phosphatidylglycerol)" and inositol, R1 and R2 can be the O-acyl-remnants of fatty acids. R2 usually is the position that binds a long chain unsaturated fatty acid like for example AA.

PL of marine origin (MPL) are extracted from water animals and preferentially from caviar, and represent glycerophospholipids with various headgroups and a high percentage of ω-3-fatty acids building the hydrophobic part. ω-3-fatty acids, namely eicosapentaenoic acid (EPA) and docosahexaenoic acid (DHA), are essential to the mammalian organism and can only be taken up by food, mainly of marine origin. Since these ω-3-fatty acids are also contained in fish oil, the supplementation of the diet with fish oil formulas is widespread in western civilizations, where the common diet is rich in meat and only little food of marine origin is consumed. Its positive effects on diseases with inflammatory background, such as rheumatoid arthritis and psoriasis are known. But, fish oil in capsules for oral supplementation, especially in high doses is not very well accepted by consumers, as it very often causes belching with fish taste or aberrations of taste and smell.

In WO2007/009819 it was moreover described that the administration of marine phospholipids has positive effects on tumor-associated weight loss in cancer patients via stimulation of appetite and reduction of inflammatory processes.

### Summary of the Invention

Surprisingly it was now found that the administration phospholipids containing ω-3-fatty acids and/or containing low amounts of ω-6 fatty acids, notably phospholipids of marine origin (MPL), to overweight or obese people leads to a reduction of appetite and body weight. Also administration of phospholipids to people with addictive behavior reduced craving for the respective substance. The invention thus provides
(1) a pharmaceutical composition or medicament comprising at least one phospholipid containing ω-3-fatty acids and/or containing no or only low amounts of ω-6 fatty acids for the treatment or prophylaxis of overweight, obesity and addictive behavior;
(2) a preferred embodiment of (1) above, wherein the at least one phospholipid is a phospholipid of marine origin (MPL);
(3) a further preferred embodiment of (1) or (2) above, wherein said at least one phospholipid is a compound represented by the formula I 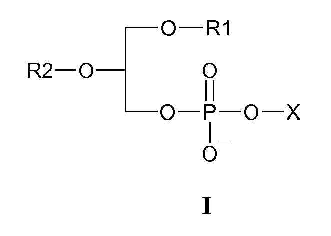 wherein
   R¹ und R² are independently selected from H and C₁₆₋₂₄ acyl residues, which may be saturated or unsaturated and may carry 1 to 3 residues R³ and wherein one or more of the C-atoms may be substituted by O or NR⁴;
   X is selected from H, -(CH₂)ₙ-N(R⁴)₃⁺, -(CH₂)ₙ-CH(N(R⁴)₃⁺)-COO⁻, -(CH₂)ₙ-CH(OH)-CH₂OH and -CH₂(CHOH)ₙ-CH₂OH, wherein n is an integer from 1 to 5;
   R³ is independently selected from H, lower alkyl, F, Cl, CN und OH; and
   R⁴ is independently selected from H, CH₃ und CH₂CH₃,
   or a pharmacologically and dietetically acceptable salt thereof;
(4) the use of a composition comprising at least one phospholipid as defined in(1) to (3) above for preparing a medicament for the treatment or prophylaxis of overweight, obesity and addictive behavior;
(5) a food supplement containing a composition comprising at least one phospholipid or MPL as defined in (1) to (3) above; and
(6) a method for the treatment or prophylaxis of overweight, obesity and addictive behavior which comprises administering a patient in need of such treatment a composition comprising at least one phospholipid as defined in (1) to (3) above.

### Short Description of the Figure

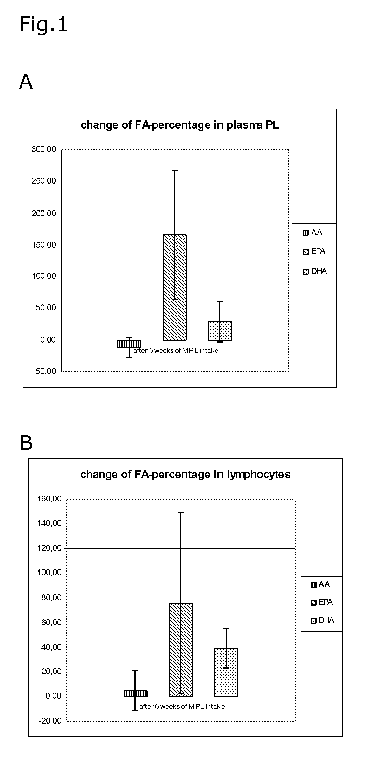

Fig. 1 shows FA-pattern changes in plasma PL (A) and whole blood lymphocytes (B) after 6 weeks of MPL intake.

### Detailed Description of the Invention

As set forth above, aspects (1) to (6) of the invention are based on the finding that the administration of phospholipids containing ω-3-fatty acids and/or containing low amounts of ω-6 fatty acids, notably phospholipids of marine origin (MPL) to overweight or obese people leads to a reduction of appetite and body weight.

The term "addictive behavior" refers to people with nicotine, alcohol, medicament, etc. addictive behavior.

The term "phospholipid" and "at least one phospholipid" include phospholipids derived from natural resources and synthetic phospholipids.
"MPL" and "phospholipids of marine origin" according to aspect (2) of the invention refer to phospholipid derived from water animals such as fish, and most preferably is isolated from fish liver or roe. Particular preferred MPL are those of aspect (3) of the invention.

It is to be understood that said at least one phospholipid or MPL of the composition or medicament according to aspects (1) to (4) and (6) of the invention contains no or only low amounts of ω-6 fatty acids, preferably no or only low amounts of arachidonic acid. Furthermore it is is to be understood that the composition or medicament contains ω-3-fatty acids, preferably eicosapentaenoic acid (EPA) or docosahexaenoic acid (DHA).

Said at least one phospholipid is selected from 1,2-diacylglycerophospholipids, 1-acylglycerophospholipids and 2-acylglycerophospholipids having saturated and/or unsaturated acyl residues or pharmacologically acceptable salts thereof. Moreover, said at least one phospholipid preferably contains acyl residues selected from saturated acyl residues, v-3- and ω-9-fatty acid residues and mixtures thereof, and the content of said acyl residues preferably is at least 75% by weight, preferably at least 90% by weight, most preferably at least 96% by weight of the total acyl residues present within the phospholipid. It is moreover preferred that said at least one phospholipid is a phosphatidylcholin.

In a preferred embodiment of the invention, said at least one phospholipid is comprised in the composition or medicament in an amount of 5 to 100% by weight, preferably 30 to 100% by weight (basis total weight of the composition or medicament).

In a further preferred embodiment of the invention, the composition or medicament further contains triglycerides, free fatty acids diglycerides and/or monoglycerides, wherein the total content of said further glycerides and fatty acids is no more than 95% by weight, preferably no more than 90% by weight, most preferably no more than 75% of the total lipids of the composition or medicament.

In another preferred embodiment of the invention, the composition or medicament contains oils, preferably fish oils.

Still in a further preferred embodiment of the invention, where said at least one phospolipid contains no or only low amounts of ω-6 fatty acids, preferably no or only low amounts of arachidonic acid, the composition or medicament further comprises an oil having a high content of ω-3-fatty acids.

In the compound of formula I according to aspect (3) of the invention it is preferred that R¹ und R² are independently selected from H and unsubstituted C₁₆₋₂₄ acyl residues, which may be saturated or unsaturated, and X is selected from a choline, serine, ethanolamine and inositol residue.

Moreover, it is particularly preferred that at least one of R1 and R2 is an ω-3-fatty acid residue having at least 20 C-atoms such as an eicosapentaenoic acid (EPA) or docosahexaenoic acid (DHA) residue or is an ω-9-fatty acid residue having at least 18 C-atoms such as an oleic acid, erucic acid or nervonic acid residue. It is also preferred that the content of ω-3- and ω-9-fatty acid residues is at least 20 %, preferably at least 35 %, most preferably at least 45 % by weight of all acyl residues of the phospholipid/MPL. Finally it is preferred that the weight ratio of ω-3-fatty acids and/or ω-9-fatty acids to ω-6-fatty acids in the MPL is at least 10 : 1, most preferably at least 15 : 1.

Particularly preferred phospholipids/MPLs of the present invention include Di-DHA-, Di-EPA-, Di-Oleyl-, EPA/DHA-, hydrated egg- and soja-phosphatidylcholines.

In a particular preferred embodiment of aspects (1) to (4) and (6) of the invention the phospholipids are the sole pharmacologically or dietetically active ingredient of the composition or medicament.

In an alternative preferred embodiment, the composition or medicament further contains pharmacologically functional compound. Such functional compounds are selected from compounds for the treatment of obesity or addictive behavior including cannabinoid-receptor antagonists such as Rimonabant (5-(4-chlorphenyl)-1-(2,4-dichlorphenyl)-4-methyl-N-piperidinopyrazol-3-carbamid) and Taranabant (N-[(1S,2S)-3-(4-chlorophenyl)-2-(3-cyanophenyl)-1-methylpropyl]-2-methyl-2-[[5-(trifluoromethyl)pyridin-2-yl]oxy]propanamide); satiation enhancers/appetite suppressants including amphetamines that stimulate the release of katecholamines or inhibit the re-uptake such as diethylpropion, mazindol, phentermin, phenylpropanolamin or the like, preferably low-dosed amphetamines; re-uptake inhibitors of noradrenalin and serotonin such as sibutramin; compounds enhancing the postprandial insulin secretion including GLP analogues such as exendin-4; compounds that attenuate nicotine withdrawal symptoms such as nicotine itself as chewing gum or patch; the NA- and dopamine reuptake inhibitor bupropion (trade name: Zyban^{®}, systematical name: (RS)-2-(tert-butylamino)-3-chloropropiophenon) and the partial ACh receptor α4β2 subtype agonist Vareniclin (trade name: Champix^{®}, systematical name: (6R, 10S)-7,8,9,10-tetrahydro-6,10-methano-6H-pyrazino[2,3-h][3]benzazepin) as well as compounds used to accompany alcohol withdrawal such as Disulfiram (trade name: Antabus^{®} systematical name: 1-diethyltiocarbamoyldisulfanyl)-N,N-diethyl-methanethioamide), Acamprosat (trade name: Campral^{®}, systematical name: 3-acetamido-1-propansulfonic acid) as well as benzodiazepines, antidepressants and neuroleptic agents and inhibitors of pancreas lipase such as orlistat that are preferably used in compositions and medicaments containing ω-3-phospholipids and do contain no or only low amounts of further triglycerides.

In a further preferred embodiment the composition or medicament additionally contains dietary fibers and swelling agents such as chitosans, cellulose derivates, Pektines, mucilages, alginates, chitin derivatives, L-carnitin or pyruvat.

The amount of such pharmacologically functional compounds preferentially may be in the range of 1 to 99% by weight relative to the phospholipids/MPLs of the composition or medicament of the invention.

Besides the phospholipids/MPLs and the pharmacologically functional compounds as defined above, the composition, medicament or food supplement of aspects (1) to (6) above may further contain pharmaceutically and dietetically acceptable carriers, binders, excipients, diluent, etc.

The composition, medicament or food supplement of aspects (1) to (6) above is preferably in the form for oral administration (e.g. in the form of a tablet, capsule or the like). In case of aspects (1) to (4) and (6) it may also be in the form for intravenous administration.

The amount of pharmaceutical compositions or medicament, or food supplement of aspects (1) to (5) to be administrative to a patient, or the amount of composition administered to a patient according to the method of aspect (6) is within the ambit of the skilled practitioner, and generally lies within the range of 1 to 100 mg of the composition per kg bodyweight per day, which corresponds to a range of 0.3 to 30 mg of the phospholipid containing ω-3-fatty acids and/or containing no or only low amounts of ω-3-fatty acids per kg bodyweight per day.

The application of phospholipids/MPLs was found to have further beneficial effects.

First, it was found that the oral administration of MPL as capsules do not cause the same side effects as fish oil capsules. Long term compliance of the formulation of 70% fish oil with 30% MPL is much higher than fish oil alone, even in cancer patients suffering from appetite loss and cachexia (see Example 1).

Second, it was found that the fatty acid pattern of cellular membranes can be altered by offering PL or their so called "lyso" form, where one of the fatty acid is cleaved enzymatically out of the PL molecule. The lyso-PL are taken up into the cellular membranes and reacylated with another fatty acid to form a new membrane PL. The uptake of fatty acids into the brain across the blood brain barrier takes place in a similar way, namely by uptake of the lyso-PL Qi K. et al.; Curr Opion Clin Nutr Metab Care 2002, 5(2): 133-138. In different tumour cells *in vitro* the supplementation of a lysoPC with a defined fatty acid, namely palmitic acid (C16:0) and heptadecaneoic acid (C17:0), in the culture medium led to a highly increased proportion of these fatty acids in the cellular membrane whilst the percentage of AA decreased (see Example 2).

Third, as to be expected from these observations, the supplementation of MPL *in vivo* was found to lead to an altered fatty acid pattern in blood plasma phospholipids, lymphocytes and erythrocytes. The percentage of DHA and EPA increases whilst the percentage of AA decreases (see Example 3). In the lymphocytes this means that the fatty acid pattern of the cellular membranes of metabolically active cells can be influenced by MPL, there is less AA bound in the cellular membranes and therefore there is less AA at disposition for the biosynthesis of lipid messengers deriving from AA, inflammation processes should be reduced. On the other hand, the administration of phospholipids, notably marine phospholipids induces a reduction of AA in cellular membranes and therefore lead to a decreased level of endogenous CB1 receptor agonists deriving from AA. As a consequence, stimulation of CB1 receptors takes place only in a limited fashion, there are less orexigenic signals and the reward system is not over stimulated anymore. The expected effect in mammals is less appetite and craving either for food and therefore facilitated weight reduction or for nicotine or alcohol and facilitated cessation. In contrast to the CB1 receptor antagonist Rimonabant which induces depression and suicidal behavior in 26 % of the patients, the intake of marine phospholipids does not have this side effect, in the current trial none of the 12 patients taking MPL showed any depressive mood disorders. Quite the contrary, decreased fatigue and improved subjectively perceived quality of life could be observed.

Fourth, another positive effect of MPL was observed regarding the blood lipid levels. LDL cholesterol decreases, while HDL cholesterol levels increase or generally elevated cholesterol levels as well as serum triglyceride levels decrease during intake of MPL. This effect on blood lipids reduces the increased risk of arteriosclerosis and associated cardiac problems in obese people.

The invention is further explained by the following examples which are, however not to be construed as limiting the invention.

### Examples

### Example 1: Compliance of MPL capsules

Regular intake of MPL capsules by cancer patients suffering from appetite loss during a period of 6 weeks is at least 92% of prescribed dose (3 capsules/day) in each individual patient and on average 95% ± 4.2 % in 5 patients. This implies a very good compliance as in all cases nearly 100% of prescribed dose is taken without further surveillance, reasons for occasional miss-outs were disease-related (e.g. nausea).

### Example 2: Change of fatty acid pattern of tumour cells in vitro

Three different tumour cell lines LNCaP, AsPC1 and Du145 (available as CRL-1740, HTB-81 and CRL-1682, respectively, from American Type Culture Collection (ATCC), Rockville, Maryland, USA) were incubated *in vitro* for 72 h in DMEM (Dulbecco's Modified Eagle Medium from Gibco Invitrogen, Germany, Ord.No. 61965-026) with addition of BSA (Bovine Serum Albumine from Sigma, Germany, Ord.No. A9418-100) supplemented with either C16:0-lysoPC (1-palmitoyl-2-hydroxy-sn-glycero-3-phosphocholine (C16:0-lysoPC) from Sigma, Germany, Ord. No. L5254) or C17:0-lysoPC (1-heptadecanoyl-2-hydroxy-sn-glycero-3-phosphocholine (C17:0-lysoPC) from Avanti Polar Lipids, USA, Ord.No. 8556676P) at a concentration of 450 µM, respectively. The amounts of palmitic acid (C16:0), heptadecanoic acid (C17:0) and arachidonic acid (AA) in percent of total fatty acid in cell membranes are shown in the following Tables 1 and 2.

**Table 1: C16:0 LPC administration**

| Cell line | Annotation | C16 (%) | AA (%) |
|---|---|---|---|
| LNCaP | without C16-LPC | 34.9 | 5.2 |
| | 72h C16-LPC | 58.2 | 1.9 |
| AsPC1 | without C16-LPC | 21.7 | 3.5 |
| | 72h C16-LPC | 65.6 | 2.3 |
| Du145 | without C16-LPC | 25.8 | 5.9 |
| | 72h C16-LPC | 47.4 | 4.5 |

**Table 2: C17:0 LPC administration**

| Cell line | Annotation | C17 (%) | AA (%) |
|---|---|---|---|
| LNCaP | without C17-LPC | 13.4 | 4.5 |
| | 72h C17-LPC | 58.8 | 2.2 |
| AsPC1 | without C17-LPC | 6.3 | 3.7 |
| | 72h C17-LPC | 57.5 | 2.3 |
| Du145 | without C17-LPC | 7.7 | 6.1 |
| | 72h C17-LPC | 54.9 | 4.8 |

### Example 3: Changes of fatty acid pattern in human blood

Average FA-pattern changes (%) in plasma PL and whole blood lymphocytes (separated out of EDTA full blood samples by solid phase extraction and gradient centrifugation) of 5 patients after 6 weeks of MPL intake (3 capsules/day). The results are shown in Fig. 1.

## Claims

1. A pharmaceutical composition or medicament comprising at least one phospholipid containing ω-3-fatty acids and/or containing no or only low amounts of ω-6 fatty acids for the treatment or prophylaxis of overweight, obesity and addictive behavior.

2. The composition or medicament of claim 1, wherein said at least one phospholipids contains
(i) no or only low amounts of ω-6 fatty acids, preferably no or only low amounts of arachidonic acid; and/or
(ii) ω-3-fatty acids, preferably eicosapentaenoic acid (EPA) or docosahexaenoic acid (DHA).

3. The composition or medicament of claim 1 or 2, wherein
(i) the pharmaceutical composition or medicament contains from 5 to 100% by weight, preferably at least 30% to 100% by weight of said at least one phopholipid; and/or
(ii) the phospholipid is derived from natural resources or is a synthetic phospholipid,
(iii) said at least one phospholipid is selected from 1,2-diacylglycerophospholipids, 1-acylglycerophospholipids and 2-acylglycerophospholipids having saturated and/or unsaturated acyl residues or pharmacologically acceptable salts thereof, and/or
(iv) said at least one phospholipid contains acyl residues selected from saturated acyl residues, ω-3- and ω-9-fatty acid residues and mixtures thereof, and the content of said acyl residues preferably is at least 75% by weight, preferably at least 90% by weight, most preferably 96% by weight of the total acyl residues present within the phospholipid; and/or
(v) said at least one phospholipid is a phosphatidylcholin; and/or
(vi) the composition or medicament further contains triglycerides, free fatty acids diglycerides and/or monoglycerides, wherein the total content of said further glycerides and fatty acids is no more than 95% by weight of the total lipids of the composition or medicament; and/or
(vii)the composition or medicament contains oils, preferably fish oils; and/or
(viii) said at least one phospolipid is as defined in claim 2(i) and the composition or medicament further comprises an oil having a high content of ω-3-fatty acids.

4. The composition or medicament of any one of claims 1 to 3, wherein said at least one phospholipid is a phospholipid of marine origin (MPL), preferably is an MPL derived from water animals such as fish, and most preferably is isolated from fish liver or roe.

5. The composition or medicament of any one of claims 1 to 4, wherein said at least one phospholipid is a compound represented by the formula I 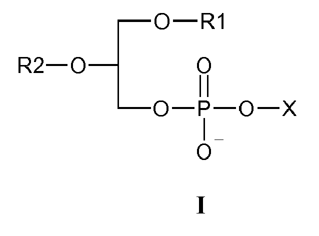 wherein
R¹ und R² are independently selected from H and C₁₆₋₂₄ acyl residues, which may be saturated or unsaturated and may carry 1 to 3 residues R³ and wherein one or more of the C-atoms may be substituted by O or NR⁴;
X is selected from H, -(CH₂)ₙ-N(R⁴)₃⁺, -(CH₂)ₙ-CH(N(R⁴)₃⁺)-COO⁻, -(CH₂)ₙ-CH(OH)-CH₂OH and -CH₂(CHOH)ₙ-CH₂OH, wherein n is an integer from 1 to 5;
R³ is independently selected from H, lower alkyl, F, Cl, CN und OH; and
R⁴ is independently selected from H, CH₃ und CH₂CH₃,
or a pharmacologically and dietetically acceptable salt thereof.

6. The composition or medicament of claim 5, wherein R¹ und R² are independently selected from H and unsubstituted C₁₆₋₂₄ acyl residues, which may be saturated or unsaturated, and X is selected from a choline, serine, ethanolamine and inositol residue, preferably
(i) at least one of R1 and R2 is an ω-3-fatty acid residue having at least 20 C-atoms such as an eicosapentaenoic acid (EPA) or docosahexaenoic acid (DHA) residue or is an ω-9-fatty acid residue having at least 18 C-atoms such as an oleic acid, erucic acid or nervonic acid residue; and/or
(ii) the content of ω-3- and ω-9-fatty acid residues is at least 20 %, more preferably at least 35 %, most preferably at least 45 % by weight of all acyl residues of the MPL; and/or
(iii) the weight ratio of ω-3-fatty acids and/or ω-9-fatty acids to ω-6-fatty acids in the MPL is at least 10 : 1, most preferably at least 15 : 1.

7. The composition or medicament of claim 5 or 6, wherein the MPL is selected from Di-DHA-, Di-EPA-, Di-Oleyl-, EPA/DHA-, hydrated egg- and soja-phosphatidylcholines.

8. The composition or medicament according to any one of claims 1 to 7, wherein
(i) the phospholipids are the sole pharmacologically or dietetically active ingredient; and/or
(ii) the composition or medicament further contains pharmacologically functional compound, preferably said further compounds being selected from compounds for the treatment of obesity including cannabinoid-receptor antagonists such as Rimonabant and Taranabant; satiation enhancers and appetite suppressants including amphetamines that stimulate the release of katecholamines or inhibit the re-uptake such as diethylpropion, mazindol, phentermin, phenylpropanolamin or the like, preferably low-dosed amphetamines; re-uptake inhibitors of noradrenalin and serotonin such as sibutramin; compounds enhancing the postprandial insulin secretion including GLP analogues such as exendin-4; and inhibitors of pancreas lipase such as orlistat that are preferably used in compositions or medicaments containing ω-3-phospholipids and do contain no or only low amounts of further triglycerides; and/or
(iii) the composition or medicament further contains dietary fibers and swelling agents such as chitosans, cellulose derivates, pektines, mucilages, alginates, chitin derivatives, L-carnitin or pyruvat; and/or
(iv) the composition or medicament is suitable for intravenous or oral administration, preferably for oral administration.

9. Use of a composition comprising at least one phospholipid according to any one of claims 1 to 6 for preparing a medicament for the treatment or prophylaxis of overweight, obesity and addictive behavior.

10. A food supplement containing a composition comprising at least one phospholipid according to any one of claims 1 to 8.

11. A method for the treatment or prophylaxis of overweight, obesity and addictive behavior, which comprises administering a patient in need of such treatment a composition comprising at least one phospholipid according to any one of claims 1 to 8.

12. The method of claim 11, wherein said composition comprises at least one MPL and/or is administered in an amount of 1 to100 mg/kg bodyweight per day.
